Europäisches Patentamt

European Patent Office

Office européen des brevets

(19)

(11) Publication number : **0 571 203 A1**

# (12) EUROPEAN PATENT APPLICATION

(21) Application number : **93303896.0**

(22) Date of filing : **19.05.93**

(51) Int. Cl.⁵ : **G01N 33/50,** C12Q 1/02, G01N 33/569

(30) Priority : **22.05.92 US 887633**

(43) Date of publication of application :
**24.11.93 Bulletin 93/47**

(84) Designated Contracting States :
**DE FR GB IT**

(71) Applicant : **Becton Dickinson and Company
One Becton Drive
Franklin Lakes New Jersey 07417-1880 (US)**

(72) Inventor : **Sussman, Mark L.
2104 Smith Avenue
Baltimore, Maryland 21209 (US)**
Inventor : **Carski, Theodore R.
14258 Baldwin Mill Road
Baldwin, Maryland 21013 (US)**

(74) Representative : **Ruffles, Graham Keith et al
MARKS & CLERK 57-60 Lincoln's Inn Fields
London WC2A 3LS (GB)**

(54) Transporting medium for microorganism specimen containing white blood cell lytic agents.

㊼ An improved transport medium contains a non-ionic detergent, preferably saponin for lysing white blood cells is used in the transport of microbiological samples from mammalian body sites. The improved transport medium contains a lytic agent, preferably saponin to quickly lyse white blood cells present in the sample, which may destroy the microorganisms and their antigenic sites. The preferred lytic agent saponin is present in a concentration from about 0.1 mg to about 10 mg, saponin/ml transport medium, preferably from about 0.5 mg up to about 2 mg saponin per ml transport medium, more preferably about 0.75 to about 2 mg saponin per ml transport medium, and most preferably in a concentration of about 1 mg saponin per ml transport medium. Furthermore, these detergent lytic agents may be combined in the transport medium with phospholipids, suitably L-a Lecithin (phosphatidylcholine) which forms mixed micelles that protect saponin. Other phospholipids include phosphatidylserine, phosphatidylinositol, phosphatidic acid, phosphatidylglycerol, phosphatidylethanolamine and sphingomyelin. If the viability of the microorganisms is to be preserved, the improved transport medium should also include suitable buffers and a carbon source to allow the survival of the microbiological sample. Preferred transport media suitable for use in the present invention include modification (by the addition of saponin) of the following media : (1) Amies Transport Medium Without Charcoal, (2) Amies Transport Medium With Charcoal, and (3) Stuart Transport Medium.

EP 0 571 203 A1

## BACKGROUND OF THE INVENTION

### 1. Field of the Invention

This invention relates to a transport medium containing a suitable lytic agent, such as saponin, for collection, delivery and delayed processing of samples from various human and animal body sites. The transport medium is useful for the purpose of isolating and identifying microorganisms from clinical samples without allowing white blood cells in the sample to destroy the microorganisms prior to testing.

### 2. Background of the Related Art

The collection, transport and delayed processing of samples from human or animal body sites is important in the testing of the samples for the presence of microorganisms associated with disease. The samples must be maintained with suitable nutrients and carbon source in the transport medium so that the microorganisms do not die or lose antigenic (chemical) viability during storage or transport of the sample for testing in the laboratory. Often, however, phagocytes (white blood cells) will destroy many of the microorganisms in the sample, so that the testing does not provide a correct indicia of the initial concentration of microorganisms originally present in the sample.

Various microbiological specimen transport media are widely used in the collection, delivery and delayed processing of samples for isolating microorganisms associated with disease. See, for example, Amies, Can. J. Public Health, 58, 296 (1967); Stuart et al., Can. J. Public Health, 45, 73 (1954); Isenberg et al., Manual of Clinical Microbiology, Lennette et al. (Eds.), 4th ed. American Society of Microbiology, Washington, D.C. (1985); Bailey et al., Diagnostic Microbiology, "Specimen Containers and their Transport", 26-27, The C.V. Mosby Co., St. Louis, Mo. (1974); and, MacFaddin, "Media for Isolation-Cultivation Identification Maintenance of Medical Bacteria", 1, Williams and Wilkins, Baltimore, Md. (1985); also see: "Manual of BBL Products and Laboratory Procedures", 6th Ed, Power et al. (Eds.), Becton Dickinson Microbiology Systems, Cockeysville, Md. (1988). The disclosure of each of these is incorporated by reference herein. These microbiological specimen transport media are widely used in hospitals, clinics, doctors' offices, other health offices and in general microbiological specimen collection and transport devices.

Accordingly, it would be desirable to provide microbiological transport media which maintain the microorganisms in the sample viable, or maintain the viability of the microorganisms' antigenic sites during storage and transport to the testing laboratory.

Saponin has been described as useful as an additive to growth media used in radiometric bacteriological culture system because its lytic action reduces background interference generated by metabolic activity of mammalian cells in blood samples, such as red cells, white cells and platelets. See for example U.S. Patent No. 3,858,045, and U.S. Patent No. 4,994,378 in which a combination of saponin and phospholipid were added to a bacterial growth medium to reduce background interference. The disclosure of both of these patents are incorporated by reference herein. Also see U.S. Patent No. 4,144,133 to Dorn et al. describing a fungal growth media containing saponin.

A recent study compared a BACTEC 660 Standard Blood culture growth medium with the same medium containing saponin. An abstract of the results of that study was reported by Jungkind et al., in AMERICAN SOCIETY OF MICROBIOLOGY-ABSTRACTS OF THE ANNUAL MEETING-1990, C-277, 390 (1990), the disclosure of which is incorporated by reference herein. Saponin was used in the culture (growth) medium to reduce background signals due to blood cell metabolism and to release phagocytized bacteria from WBCs, resulting in improved detection of microorganisms. The results of this study are described in greater detail in Examples 1 and 2.

A bacteriological culture system using saponin is described in U.S. Patent No. 4,131,512. Saponin is used for its lytic action allowing microbial pathogens to be separated from the blood sample by centrifugation. The released pathogens pass out of suspension during centrifugation of the sample and collect in a layer adjacent to an interface between a cushioning agent and the blood sample, or enter the cushioning agent. The microbial pathogens can be isolated in and on the cushioning agent for further culturing and analysis.

A method for rapid detection of bacterial and fungal infection using saponin to rupture phagocytes is described in U.S. Patent No. 5,043,267. The method relates to a pathogen which is phagocytosed and subject to at least partial degradation by phagocytes of the host. The method includes isolating from the host test sample which contains a cellular population comprising phagocytes; contacting the population with saponin capable of rupturing the phagocytes to release at least one soluble component of the pathogen, but not capable of rupturing unphagocytosed pathogen; separating at least one soluble component of the degraded pathogen from unphagocytosed pathogen; contacting a soluble component with a biospecific binding partner for that component; and measuring the extent of binding. There is no disclosure in that patent for a composition for a transport medium.

An in vitro diagnostic technique utilizing saponin to degrade the viscosity of sputum samples and disperse microbiological pathogens uniformly in the sample is described in U.S. Patent No. 4,053,363. This patent discloses that saponin is a powerful hemolytic agent (red blood cell lytic agent) which dissolves red blood corpuscles without effecting white cell counts (see column 3, lines 16-23).

A growth medium containing saponin is used in growing of microorganisms for laboratory diagnosis of continuous ambulatory peritoneal dialysis effluent as reported by Taylor et al., "Increased Microbial Yield from Continuous Ambulatory Peritoneal Dialysis Peritonitis Effluent after Chemical or Physical Disruption of Phagocytes", Journal of Clinical Microbiology, 25(3), 580-583 (1987). Taylor et al. reported that clinical peritonitis effluent specimens were delivered to the laboratory in cooled dialysis bags and sonicated prior to culturing on saponin-containing growth media. The process resulted in growth of significantly greater numbers of colonies than standard culturing on conventional media. Also see: Zierdt "Simplified Lysed-Blood Culture Technique", Journal of Clinical Microbiology, 23(3), 452-455 (1986) describing a system in which a lytic agent such as Tween 20, the saponin digitonin, other polyoxyethylene adducts, or Triton X is added to the blood culture medium to promote hemolysis with minimal toxic effect to bacteria in the blood culture.

In summary, none of the related art describes the composition for a transport medium containing saponin, or the use of saponin in a transport medium in appropriate concentration to lyse phagocytes while maintaining the survivability of microorganisms or the viability of their antigenic sites in the sample.

Accordingly, a desired purpose of the present invention is to provide transport media containing components which maintain the survivability of microbiological specimens while preventing white blood cells in the media from attacking and destroying the microbiological specimens.

A further objective is to provide transport media which maintain viability of microorganisms' antigenic sites in a sample.

## SUMMARY OF THE INVENTION

These and other objectives are achieved by the present invention which provides an improved transport medium containing a non-ionic lytic agent, preferably saponin for lysing white blood cells. The improved transport medium contains the preferred lytic agent saponin to quickly lyse white blood cells present in the sample which may destroy the microorganisms and their antigenic sites. The preferred lytic agent saponin is present in a concentration from about 0.1 mg to about 10 mg, saponin/ml transport medium, preferably from about 0.5 mg up to about 2 mg saponin per ml transport medium, more preferably about 0.75 to about 2 mg saponin per ml transport medium, and most preferably in a concentration of about 1 mg saponin per ml transport medium. Furthermore, these detergent lytic agents may be combined in the transport medium with phospholipids, suitably L-a Lecithin (phosphatidylcholine) which forms mixed micelles that protect saponin. Other phospholipids include phosphatidylserine, phosphatidylinositol, phosphatidic acid, phosphatidylglycerol, phosphatidylethanolamine and sphingomyelin. If the viability of the microorganisms is to be preserved the improved transport medium should also include suitable buffers and a carbon source to allow the survival of a microbiological sample. Preferred transport media suitable for the present invention include saponin modified: (1) Amies Transport Medium Without Charcoal, (2) Amies Transport Medium With Charcoal, and (3) Stuart Transport Medium.

For a better understanding of the present invention reference is made to the following description and examples, taken in conjunction with the accompanying tables, the scope of which is pointed out in the appended claims.

## DETAILED DESCRIPTION OF THE INVENTION

The present invention provides an improved transport medium containing a non-ionic detergent, preferably saponin for lysing white blood cells used for the transport of microbiological samples from mammalian body sites. The improved transport medium contains the preferred lytic agent saponin to quickly lyse phagocytes (white blood cells) present in the sample which may destroy the microorganisms and their antigenic sites. The preferred lytic agent saponin is present in a concentration from about 0.1 mg saponin to about 10 mg saponin per ml transport medium, preferably in a concentration from about 0.5 mg to about 2 mg saponin per ml transport medium, more preferably from about 0.75 to about 1 mg saponin per ml transport medium, and most preferably in a concentration of about 1 mg saponin per ml transport medium. If the improved transport media is only required for maintaining the integrity of the antigenic sites of the released microorganisms, then a carbon source and buffers are not required.

If the viability of the microorganisms is to be preserved, the improved transport media should also include suitable buffers and a carbon source to allow the survival of the microbiological sample. Preferably, the saponin is added to suitable transport media formulations as described in Amies, Can. J. Public Health, 58, 296 (1967); Stuart et al. Can. J. Public Health, 45, 73 (1954); (Isenberg Manual of Clinical Microbiology, Lennette et al. (Eds.), 4th ed. ASM, Washington, D.C. (1985); Bailey et al. Diagnostic Microbiology : "Specimen Containers and their Transport", 26-27, The C.V. Mosby Co., St. Louis, Mo. (1974) ; and, MacFaddin, "Media for Isolation-Cultivation Identification Maintenance of Medical Bacteria", 1, Williams and Wilkins, Baltimore, Md. (1985); also see Manual of BBL Products and Laboratory Procedures", 6th Edition, Power et al. (Eds.) Becton Dickinson Microbiology Systems, Cockeysville, Md. (1988), the disclosures of which are incorporated by reference herein. Three preferred transport media suitable for modification with saponin, as discussed above, for use with the present invention include:

(1) Amies Transport Medium Without Charcoal, containing:

| | |
|---|---|
| Sodium thioglycolate | 1.00 g |
| Sodium Chloride | 3.00 g |
| Potassium chloride | 0.20 g |
| Calcium chloride | 0.10 g |
| Magnesium chloride | 0.10 g |
| Potassium phosphate, monobasic | 0.20 g |
| Sodium Phosphate, dibasic | 1.15 g |
| Agar | 4.00 g |
| Demineralized Water | 1 liter |

**Final pH 7.4 +/-0.2 at 25°C.**

(2) Amies Transport Medium With Charcoal, containing the above ingredients and 10 g of charcoal per liter.

(3) Stuart Transport Medium, containing:

| | |
|---|---|
| Sodium thioglycolate | 1.0 g |
| Sodium glycerophosphate | 10.0 g |
| Calcium chloride | 0.1 g |
| Methylene blue | 0.002 g |
| Agar | 3.0 g |
| Demineralized water | 1 liter |

**Final pH 7.3 +/-0.2 at 25°C**

The improved formulation of the present invention provides rapid lysis of white blood cells ("WBC's") thus freeing phagocytized microorganisms in the sample, and preventing WBC's from attacking free microorganisms. The release of the phagocytized microorganisms is desirable because of the environment within the white blood cells is hostile (inhibitor or lethal) to the microorganisms. Freeing the microorganisms from this environment releases and places the microorganisms in a favorable environment provided by the transport medium, which is designed to preserve the viability of microorganisms, or at least their antigenic sites, but not stimulate growth.

In addition to saponin, a suitable non-ionic detergent lytic agent, other non-ionic detergents may be utilized in the present invention, such as sodium cholate, Tween 20, some of the Triton X series, Brijs 56, 58, 76, 78, 96, 99, lysolecithin and polyoxyethylene-10-tridecyl ether. A detergent concentration from about 0.02% to about 2.0% is suitable. In addition, these detergent lytic agents may be combined, preferably in approximately equal proportions, in the transport medium with phospholipids, suitably L-a Lecithin (phosphatidylcholine) which forms mixed micelles that protect saponin, as described in U.S. Patent No. 4,994,378, the disclosure of which is incorporated by reference herein. Other phospholipids described in that patent which may be used in the present invention include phosphatidylserine, phosphatidylinositol, phosphatidic acid, phosphatidylglycerol, phosphatidylethanolamine and sphingomyelin.

The following examples further illustrate the various features of the present invention.

## EXAMPLES

Saponin is known to lyse red blood cells in culture medium, however, it was not clear whether saponin's activity extends to lysing white blood cells. In fact, previous studies, for example U.S. Patent No. 4,053,363 column 3 at lines 16-23, indicated that saponin may not be capable of lysing white blood cells. Accordingly, the following experiments were performed, although Examples 1 and 2 utilized culture (growth) medium rather than transport medium, in order to determine whether saponin has the potential for lysing white blood cells in transport medium as described in Example 3.

In a recent study a BACTEC 660 Standard Blood culture (growth) medium was compared with the same medium containing saponin. An abstract of the results of that study was reported by Jungkind et al., in AMERICAN SOCIETY OF MICROBIOLOGY-ABSTRACTS OF THE ANNUAL MEETING-1990, C-277, 390 (1990). In that study saponin was used in the culture (growth) medium to reduce background signal due to blood cell metabolism and to release phagocytized bacteria for WBCs, resulting in improved detections of microorganisms. The results of this study are described in greater detail in Examples 1 and 2.

## Example 1

This example tests white cell lysis in anaerobic medium containing 40mg/ml saponin, Becton Dickinson.

Two samples of anaerobic culture (growth) media were tested, one with saponin and one without. The saponin free medium is commercially available from Becton Dickinson, Cockeysville, Maryland as BACTEC® anaerobic culture medium NR-7A, Catalog No. 04392 in MA-0034, PP-044D, published by Becton Dickinson, (June 1989), containing the following ingredients:

Table 1

| BACTEC® NR7A Anaerobic Culture Medium | |
|---|---|
| Constituents | Quantity |
| Processed Water | 30 ml |
| Soybean-Casein Digest Broth | 2.75% w/v |
| Hemin | 0.0005% w/v |
| Vitamin K | 0.00005% w/v |
| Dextrose | 0.2% w/v |
| Sodium Polyanetholesulfonate (SPS) | 0.035% w/v |
| Yeast Extract | 0.20% w/v |
| Animal Tissue Digest | 0.05% w/v |
| Sodium Citrate | 0.02% w/v |
| Thiols | 0.10% w/v |

Two (2) Bottles containing 30 ml of the BACTEC® anaerobic culture medium NR7A were compared with two identical samples of BACTEC® culture medium NR7A, which also contained 40 mg saponin per bottle (a total of four bottles). Blood from donor I (See Table 2) was placed in each bottle. Duplicate counting of samples for each data point drawn was performed. The bottles containing saponin were designated as samples A and C, and the control media not containing saponin were designated as samples B and D. Donor I was a healthy young adult caucasian male, the results of the blood tests on donor I are tabulated in Table 2, as follows:

## Table 2

| Blood Fraction | Quantity | WBC DIFF | (%) |
|---|---|---|---|
| White Blood Cells | $6.8 \times 10^3/mm^3$ | Neutrophil | 51.0 |
| Red Blood Cells | $4.51 \times 10^6/mm^3$ | Lymphocytes | 36.9 |
| Hemoglobin | 15.3 g/dl | Monocytes | 6.8 |
| Hematocrit | 44.1% | Eosinophil | 1.3 |
| Platelets | $247.0 \times 10^3/mm^3$ | Basophil | 1.1 |
| Serum total cholesterol | 226 mg/dl | *Luc | 2.8 |

*Large unclassified cells

The blood from Donor I (40 ml) was collected in two large yellow-top VACUTAINER tubes. Each bottle of medium was warmed to 35°C and four (4) ml of blood was injected with a hypodermic needle through the septum of each bottle using aseptic technique. The white blood cells from 30 ml of blood from Donor I were harvested by collection of the leucocyte rich fraction after the red blood cells (RBC's) settled out at 1 X gravity. The white blood cells were separated and labeled with Indium[111], and an equal quantity of the cells representing WBC's from approximately 4 ml of whole blood were added to each sample bottle A-D. This effectively doubled the WBC count in each sample.

The percent of lysis of WBC's in these media was tested by determining the quantity of Indium[111] released into the media by using a radioactivity counter. All counts were made in duplicate and averaged. The background count of 200 cpm was subtracted from these counts. Table 3 shows the percent lysis of WBC's in the saponin containing and control anaerobic medium NR7A.

Table 3

| Percent Lyses of WBC in Control and Saponin Containing Anaerobic Culture Media | | | | |
|---|---|---|---|---|
| | Sample | | | |
| Time | Saponin A | Saponin C | Control B | Control D |
| 30 sec. | 95.3% | 84.5% | 2.3% | 2.2% |
| 15 min. | 100.9% | 92.5% | 2.4% | 2.7% |
| 30 min. | 102.4% | 95.3% | 2.9% | 6.6% |
| 60 min. | 109.7% | 98.7% | 4.5% | 6.7% |
| 2 hr. | 101.1% | 96.6% | 4.6% | 5.1% |
| 4 hr. | 102.4% | 97.3% | 6.6% | 7.2% |
| 21 hr. | 104.9% | 96.8% | 18.6% | 19.0% |

Table 4 shows the release of Indium[111] from white blood cells in the control anaerobic culture medium and saponin containing anaerobic culture medium samples, as measured in cpm's for each 0.5 ml sample. The total radioactivity in counts per minute ("cpm") for each 0.5 ml sample aliquot was 143379 cpm for Sample A, 142137 cpm for Sample C, 144820 cpm for Sample B, and 137932 cpm for Sample D (designated as STD in Table 4).

6

Table 4

| Release of Indium[111] from WBC in anaerobic control medium and saponin containing samples. | | | | |
|---|---|---|---|---|
| | CPM'S per Each 0.5 ml Sample | | | |
| Time | Saponin A | Saponin C | Control B | Control D |
| 30 sec. | 127284 | 121447 | 3521 | 3166 |
| | 146429 | 119286 | 3614 | 3312 |
| 15 min. | 135617 | 129206 | 3652 | 3865 |
| | 154189 | 134281 | 3812 | 4036 |
| 30 min. | 136139 | 131247 | 4335 | 8991 |
| | 157886 | 140065 | 4548 | 9487 |
| 60 min. | 140409 | 130691 | 5412 | 9319 |
| | 174632 | 150213 | 7906 | 9515 |
| 2 hr. | 138725 | 132276 | 6801 | 7210 |
| | 151641 | 142594 | 7027 | 7381 |
| 4 hr. | 150527 | 136645 | 9632 | 9979 |
| | 143628 | 140345 | 9917 | 10304 |
| 21 hr. | 149885 | 141233 | 26337 | 25977 |
| | 151291 | 134229 | 27897 | 26853 |
| STD | 146949 | 142036 | 148818 | 139623 |
| | 140209 | 142637 | 141221 | 136641 |

The figures shown in Table 4 were not corrected for the 200 cpm background reading.

This example clearly shows that anaerobic medium containing at least a one (1) mg saponin per ml of anaerobic medium is highly effective in lysing white blood cells. Moreover, between 84% and 95% of the cells were lysed in the first 30 seconds, while from 90% to 100% of the cells were lysed in the first 15 minutes.

Example 2

The example was performed in order to determine whether saponin in the same concentration (i.e., 1 mg saponin per ml of the culture (growth) medium), as tested in Example 1, as well as of lower concentrations is equally effective for lysing white blood cells in aerobic culture (growth) medium as it was in the anaerobic culture (growth) medium described in Example 1.

Five (5) bottles containing BACTEC® aerobic culture medium NR-6A commercially available from Becton Dickinson, Cockeysville, Maryland Catalog No. 04391, MA-0034, PP 044D (June 1989), containing the ingredients listed in Table 5 were tested.

Table 5

| BACTEC®NR6A Anaerobic Culture Medium® | |
|---|---|
| Constituents | Quantity |
| Processed Water | 30 ml |
| Soybean-Casein Digest Broth | 2.75% w/v |
| Hemin | 0.0005% w/v |
| Vitamin K | 0.00005% w/v |
| Dextrose | 0.06% w/v |
| Sucrose | 0.0835% w/v |
| Sodium Polyanetholesulfonate (SPS) | 0.035% w/v |
| Antifoaming Agent | 0.01% w/v |
| Pyridoxal HCl (Vitamin $B_6$) | 0.001% w/v |

The aerobic culture medium described in Table 5 was divided into five (5) BACTEC® culture bottles (designated A-E) containing 30 ml medium per bottle. The first bottle was designated as the control (A) sample which did not contain saponin; to the second bottle 20 mg saponin was aseptically added for a concentration of 0.5 mg saponin/ml aerobic culture medium and designated as Sample (B); to the third bottle 30 mg saponin was aseptically added for a concentration of 0.75 mg saponin/ml aerobic culture medium and designated as Sample (C); and to the remaining two (2) bottles 40 mg of saponin was aseptically added for a concentration of 1.0 mg. saponin/ml aerobic culture medium and designated as Samples (D) and (E), respectively. For this example, duplicate counting of samples for each data point was performed. The blood from Donor II, a healthy middle-age adult caucasian male was tested. The results of the blood tests are listed in Table 6, as follows:

Table 6

| Blood Fraction | Quantity | WBC DIFF | (%) |
|---|---|---|---|
| White Blood Cells | $5.01 \times 10^3/mm^3$ | Neutrophil | 46.4 |
| Red Blood Cells | $4.34 \times 106/mm^3$ | Lymphocytes | 41.8 |
| Hemoglobin | 14.3 g/dl | Monocytes | 6.5 |
| Hematocrit | 39.5 % | Eosinophil | 2.4 |
| Platets | $220 \times 103/mm^3$ | Basophil | 0.6 |
| Serum total cholesterol | 290 mg/dl | *Luc | 2.3 |

*Large unclassified cells.

The blood from donor II (40 ml) was collected in two large yellow-top VACUTAINER tubes. Each bottle of media was warmed to 35°C and four (4) ml of blood was injected with a hypodermic needle through the septum of each bottle using aseptic technique. The white blood cells from 30 ml of blood from donor II were harvested by collection of the leucocyte rich fraction after the red blood cells (RBC's) settled out at 1 X gravity. The white blood cells were separated and labeled with Indium[111], and equal quantity of the cells representing WBC's from approximately 4 ml of whole blood were added to each sample bottle A-E. This provided the effect of doubling the WBC count in the same manner as described in Example 1.

The percent lysis of WBC's in these media was tested by determining the quantity of Indium[111] released into the medium, using the same radioactivity count described in Example 1. Table 7 shows the percent lysis of WBC's in the saponin containing aerobic culture media B through E, and control anaerobic culture medium A not containing saponin.

Table 7

| Percent Lyses of WBC in Control and Saponin Containing Aerobic Media | | | | | |
|---|---|---|---|---|---|
| | Sample | | | | |
| Time | Control A | Saponin B | Saponin C | Saponin D | Saponin E |
| 30 Sec. | 2.8% | 36.8% | 72.8% | 84.6% | 88.5% |
| 15 min. | 3.7 | 55.1 | 84.6 | 92.6 | 92.5 |
| 30 min. | 4.2 | 58.7 | 87.3 | 92.3 | 92.1 |
| 60 min. | 5.0 | 66.9 | 86.3 | 91.5 | 93.7 |
| 2 hr. | 6.5 | 71.3 | 92.0 | 91.4 | 94.4 |
| 4 hr. | 7.7 | 76.1 | 90.8 | 91.0 | 95.2 |
| 21 hr. | 14.6 | 87.3 | 93.8 | 95.4 | 98.5 |

The background count of 200 cpm was not subtracted for these tabulations.

Table 8 shows the release of Indium[111] from white blood cells in the control aerobic medium (A) and saponin containing aerobic culture media samples B-E, as measured in cpm's for each 0.5 ml sample. The total radioactivity in counts per minute ("cpm") for each 0.5 ml sample aliquot tested was 36415 cpm for sample A, 34945 cpm for sample B, 36700 cpm for sample C, 36388 cpm for sample D and 33553 cpm for sample E (designated as STD in Table 8).

Table 8

| | CPM's per Each 0.5 ml Sample | | | | |
|---|---|---|---|---|---|
| Time | Control A | Saponin B | Saponin C | Saponin D | Saponin E |
| 30 Sec. | 1093 | 12938 | 26453 | 29276 | 29394 |
| | 952 | 13339 | 26946 | 32093 | 29980 |
| 15 min. | 1294 | 19178 | 30521 | 33265 | 30989 |
| | 1382 | 19301 | 31585 | 33994 | 31083 |
| 30 min. | 1520 | 20592 | 31635 | 33250 | 30334 |
| | 1525 | 20454 | 32404 | 33909 | 31468 |
| 60 min. | 1771 | 23374 | 31731 | 33405 | 30659 |
| | 1893 | 23363 | 31596 | 33165 | 32225 |
| 2 hr. | 2214 | 24664 | 33835 | 34080 | 31081 |
| | 2554 | 25138 | 33682 | 32879 | 32265 |
| 4 hr. | 2719 | 26625 | 32770 | 32453 | 31405 |
| | 2876 | 26588 | 33877 | 33738 | 32502 |
| 21 hr. | 5265 | 30097 | 34164 | 34439 | 32475 |
| | 5347 | 30939 | 34711 | 35021 | 33628 |
| STD | 36006 | 34467 | 36519 | 35577 | 32853 |
| | 30823 | 35432 | 36879 | 37198 | 34253 |

Release of Indium[111] from WBC in aerobic control medium and saponin containing samples.

The figures shown in Table 8 were not corrected for the 200 cpm background reading.

This Example shows that the aerobic culture medium containing 0.5 mg saponin per ml of aerobic culture medium lysed over 55% of the WBC's in the first 15 minutes. Moreover in the aerobic culture medium containing 0.75 mg of saponin per ml of aerobic culture medium, over 70% of the white blood cells were lysed in the first 30 seconds and more than 84% of white blood cells were lysed within the first 15 minutes. In the aerobic culture medium containing 1 mg saponin per ml of aerobic culture medium, over 84% of the white blood cells were lysed in the first 30 seconds and over 92% of the white blood cells were lysed in the first 15 minutes. These results compare favorably with 1 mg of saponin per ml of anaerobic culture medium which was tested in Example 1.

Example 3

Transport medium is prepared and divided into two aliquots, A and B. Suitable formulations for transport medium are described by Stuart et al., Can. J. of Public Health, 45, 73 (1954); and Amies, Can. J. of Public Health, 58, 296 (1967). In aliquot A, saponin (a solid) is added in a sufficient quantity to make the final concentration in the transport medium to be 1 mg saponin/ml of transport medium. Aliquot B is maintained as a saponin-free control. One hundred (100) tubes are prepared for each aliquot. The media are then aliquotted to each of the tubes (2 ml./tube) and autoclaved for sterility.

In a clinical setting one hundred (100) exudative wound cultures are sampled using two swabs each. Each swab is then placed into either medium A or B in a randomized fashion. The tubes are then transported to a clinical microbiology laboratory for culture and testing.

In a microbiology laboratory each swab is thoroughly rinsed in 1.0 ml physiological saline. One tenth (1/10) ml aliquots are placed onto and evenly spread across the surface of each of four agar media: (1) Tryptic soy agar with 5% sheep blood, (2) MacConkey Agar, (3) CNA Agar, and (4) Chocolate Agar. The plates are then placed in a suitable incubator at 37°C for 48 hours. At the end of the incubation time the plates are removed and the number of colonies in each plate are enumerated. The enumeration of colonies is done in parallel for each of the samples tested in transport medium A with saponin, and for the control transport medium B without saponin.

Results from colony enumeration for each medium pair from transport media A and B are compared by appropriate statistical comparison, such as the Chi Square Test. The culture plates swabbed from transport medium A are expected to provide greater bacterial recovery due to lysis of white blood cells, and release of bacteria from the harsh bactericidal conditions internal to phagocytes.

Example 4

Transport medium is prepared and divided into two aliquots, A and B as described in Example 5. In aliquot A, saponin (a solid) is added in a sufficient quantity to make the final concentration in the transport medium to be 1 mg saponin per ml of transport medium. Aliquot B is maintained as a saponin free control. One hundred (100) tubes are prepared for each aliquot. The media are then autoclaved for sterility.

In clinical setting one hundred (100) throat cultures are sampled using two swabs each. Each swab is then placed into either medium A or B in a randomized fashion. The tubes are then transported to a clinical microbiology laboratory for antigenic testing.

In a microbiology laboratory each swab is subjected to appropriate preparatory procedures to perform a screening test for Group A Streptococci (S. pyogenes). An example of such a test is the BBL Directigen® Group A Strep test (Becton Dickinson Microbiology Systems, Cockeysville, MD 21030).

Results from each test pair from transport media A and B are compared by appropriate statistical comparison, such as a Student T Test. The serological test using swabs from transport medium A (with saponin) are expected to provide greater antigenic recovery due to lysis of white blood cells, and release of bacteria from the harsh bactericidal conditions internal to phagocytes.

Thus, while there have been described what are the presently contemplated preferred embodiments of the present invention, further changes and modifications could be made by those skilled in the art without departing from the spirit and scope of the invention, and it is contemplated to claim all such changes and modifications.

## Claims

1. An improved transport medium for maintaining the viability of a microbiological specimen which includes suitable buffering agents in a carbon source to support the viability of microorganisms, the improvement comprises:

   an effective amount of a white blood cell lytic agent capable of releasing microorganisms from phagocytic cells.

2. An improved transport medium as recited in claim 1, wherein the said lytic agent is a non-ionic detergent or saponin.

3. An improved transport medium as recited in claim 1, wherein said lytic agent is saponin, and wherein said improvement further comprises a phospholipid selected from the group consisting of L-a Lecithin, phosphatidylserine, phosphatidylinositol, phosphatidic acid, phosphatidylglycerol, phosphatidylethanolamine, sphingomyelin, and mixtures thereof.

4. An improved transport medium for maintaining the integrity of antigenic sites of a microbiological specimen, wherein the improvement comprises an effective amount of a white blood cell lytic agent capable of releasing intact antigenic sites of microorganisms from phagocytic cells.

5. An improved transport medium as recited in claim 4, wherein the said lytic agent is a non-ionic detergent or saponin.

6. A method for transporting a microbiological specimen comprising:

   contacting a microbiological specimen with a transport medium which includes suitable buffering agents and a carbon source to support the viability of a microorganism and an effective amount of a white blood cell lytic agent capable of releasing microorganisms from phagocytic cells; and

maintaining said microbiological specimen in said transport medium until said specimen is released for use or testing,

whereby said transport medium releases microorganisms from white blood cells which may destroy the microorganisms and supports the viability of said microbiological specimen.

7.    A method as recited in claim 6, wherein the said lytic agent is a non-ionic detergent or saponin.

8.    A method for transporting a microbiological specimen comprising:

contacting a microbiological specimen with a transport medium which includes an effective amount of a white blood cell lytic agent capable of releasing intact antigenic sites of microorganisms from phagocytic cells; and

maintaining said microbiological specimen in said transport medium until said specimen is released for use or testing.

9.    A method as recited in claim 8, wherein the said lytic agent is a non-ionic detergent or saponin.

European Patent Office

**EUROPEAN SEARCH REPORT**

Application Number

EP 93 30 3896

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | JOURNAL OF CLINICAL MICROBIOLOGY vol. 25, no. 3, March 1987, pages 580 - 583. P.C.TAYLOR ET AL. 'Increased Microbial Yield from Continuous Ambulatory Peritoneal Dialysis Peritonitis Effluent after Chemical or Physical Disruption of Phagocytes.' * the whole document * | 1,2 | G01N33/50 C12Q1/02 G01N33/569 |
| A,D | JOURNAL OF CLINICAL MICROBIOLOGY vol. 23, no. 3, March 1986, pages 452 - 455 C.H.ZIERDT 'Simplified Lysed-Blood Culture Technique.' * page 452 - page 453 * | 1,2 | |
| Y,D | US-A-5 043 267 (J.C.RICHARDS) * the whole document * | 1-9 | |
| Y,D | US-A-4 994 378 (D.M.BERGER ET AL.) * abstract; claims * | 1-9 | TECHNICAL FIELDS SEARCHED (Int. Cl.5 ) |
| Y,D | CANADIAN JOURNAL OF PUBLIC HEALTH vol. 58, no. 7, July 1967, pages 296 - 300 C.R.AMIES ET AL. 'A Modified Formula for the Preparation of Stuart's Transport Medium.' * the whole document * | 1-9 | G01N C12Q C12N |
| A,D | Tha American Society of Microbiology Abstracts of the Annual Meeting - 1990 Page 390. Abstract No. C-277. D.L.Jungkind et al. *The Abstract. * | 1-9 | |

The present search report has been drawn up for all claims

| Place of search | Date of completion of the search | Examiner |
|---|---|---|
| THE HAGUE | 30 AUGUST 1993 | HITCHEN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................
& : member of the same patent family, corresponding document

EPO FORM 1503 03.82 (P0401)